# EUROPEAN PATENT APPLICATION

(11) **EP 0 829 477 A1**
(43) Date of publication of application: **18.03.1998**
(21) Application number: 96912266.2
(22) Date of filing: 26.04.1996
(51) Int. Cl.: C07D 311/36, C12P 17/16, A61K 31/35

(54) **NOVEL PHYSIOLOGICALLY ACTIVE NA23063 ANALOGUES, PROCESS FOR PRODUCING THE SAME AND USE OF THE SAME**

(30) Priority: 09.05.1995 JP 134705/95
(71) Applicant: NIPPON KAYAKU KABUSHIKI KAISHA, Tokyo 102 (JP)
(72) Inventor: NISHIKIORI, Takaaki, Yono-shi, Saitama 338 (JP); TSUCHIYA, Kouichi, Yono-shi, Saitama 338 (JP); KOBAYASHI, Shinichi, Ageo-shi, Saitama 362 (JP); HARADA, Takashi, Outa-ku, Tokyo 144 (JP); ISHIDA, Kouichi, Mitsukaido-shi, Ibaraki 303 (JP)
(74) Representative: Türk, Gille, Hrabal, Leifert
(86) International application number: JP9601163
(87) International publication number: WO9635683

(57) **Abstract**

Novel physiologically active NA23063 analogues and pharmacologically acceptable salts thereof. Microorganisms belonging to the genus Streptomyces and capable of producing physiologically active substances NA23063A, B1, B2, C and D were incubated in a medium and the physiologically active substances NA23063A, B1, B2, C and D thus accumulated in the culture were collected. These compounds were further converted into derivatives thereof. For example, the structure of NA23063D is given below.

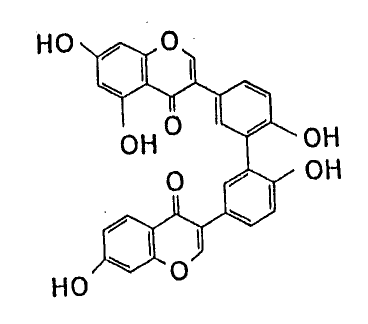

Because of having the effect of inhibiting phosphodiesterases, the compounds are useful as the active ingredient of, for example, an antiasthmatic agent, a bronchodilator, a remedy for bronchitis, an antiallergic agent, an anti-inflammatory agent, an antirheumatic agent, a hypotensive drug, a remedy for angina pectoris, a remedy for arrhythmia, a cerebral vasodilator, a blood coagulation inhibiting agent or an antidepressant agent.

## Description

### TECHNICAL FIELD

The present invention relates to a process for producing novel physiologically active NA23063 analogues, and to use of the analogues. The compounds of the invention have the effect of inhibiting phosphodiesterases, and are expected to be physiologically active substances usable, for example, as antiasthmatic agents, bronchodilators, remedies for bronchitis, antiallergic agents, antiinflammatory agents, antirheumatic agents, hypotensive drugs, remedies for angina pectoris, remedies for arrhythmia, cerebral vasodilators, blood coagulation inhibiting agents and antidepressant agents.

### BACKGROUND ART

As physiologically active substances having the effect of inhibiting phosphodiesterases and usable as bronchodilators, remedies for angina pectoris, remedies for arrhythmia, cerebral vasodilators and antidepressant agents, for example, theophylline, amrinone, and rolipram have heretofore been known.

However, these compounds have strong side effects and are not satisfactory. Novel compounds having the effect of inhibiting phosphodiesterases are desired.

### DISCLOSURE OF THE INVENTION

We, the present inventors have studied various metabolites of microorganisms and, as a result, have found that one strain belonging to actinomycetes produces physiologically active substances NA23063A, B1, B2, C and D having the effect of inhibiting phosphodiesterases.

Based on that finding, the inventors have further studied and, as a result, have found that NA23063 analogues of the following general formula (1) and their pharmaceutically acceptable salts have the effect of inhibiting phosphodiesterases. 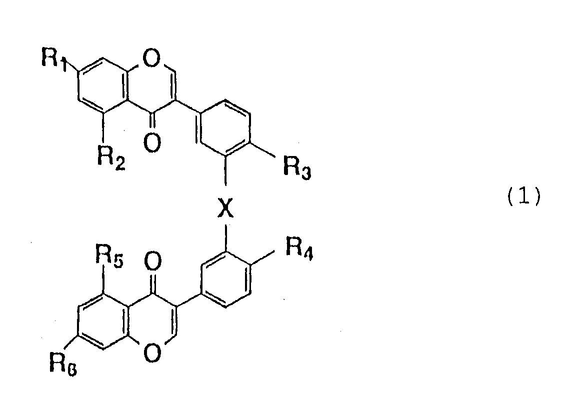 Wherein R₁ to R₆ are the same or different substituents, and each independently represent a hydrogen atom, a hydroxyl group or OR; R is the same group and represents a C₁₋₄ lower alkyl group, -(CH₂)ₙCOOH, -(CH₂)ₙNYY, a C₁₋₄ lower alkylcarbonyl group, -CO(CH₂)ₙCOOH or -CO(CH₂)ₙNYY; n represents an integer of from 1 to 4; Y represents a hydrogen atom or a C₁₋₄ lower alkyl group, and plural Y's may be the same or different; X represents an oxygen atom or a direct bond.

The invention has been completed on the basis of the above-mentioned findings.

The physiologically active substances NA23063A, B1, B2, C and D are obtained by cultivating microorganisms belonging to the genus Streptomyces and capable of producing NA23063A, B1, B2, C and D to make them produce those compounds, followed by collecting the compounds thus accumulated in the culture.

Structural formulas of the physiologically active substances NA23063A, B1, B2, C and D are as follows:

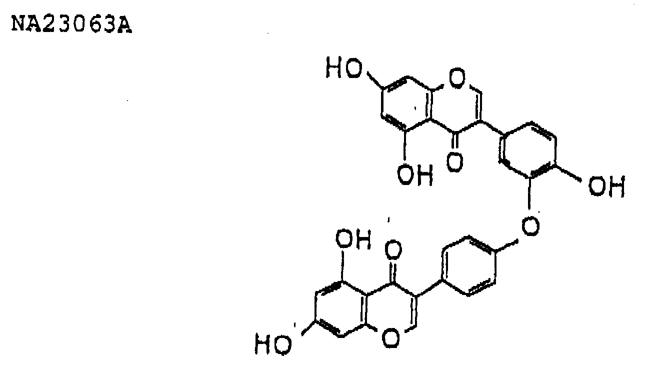

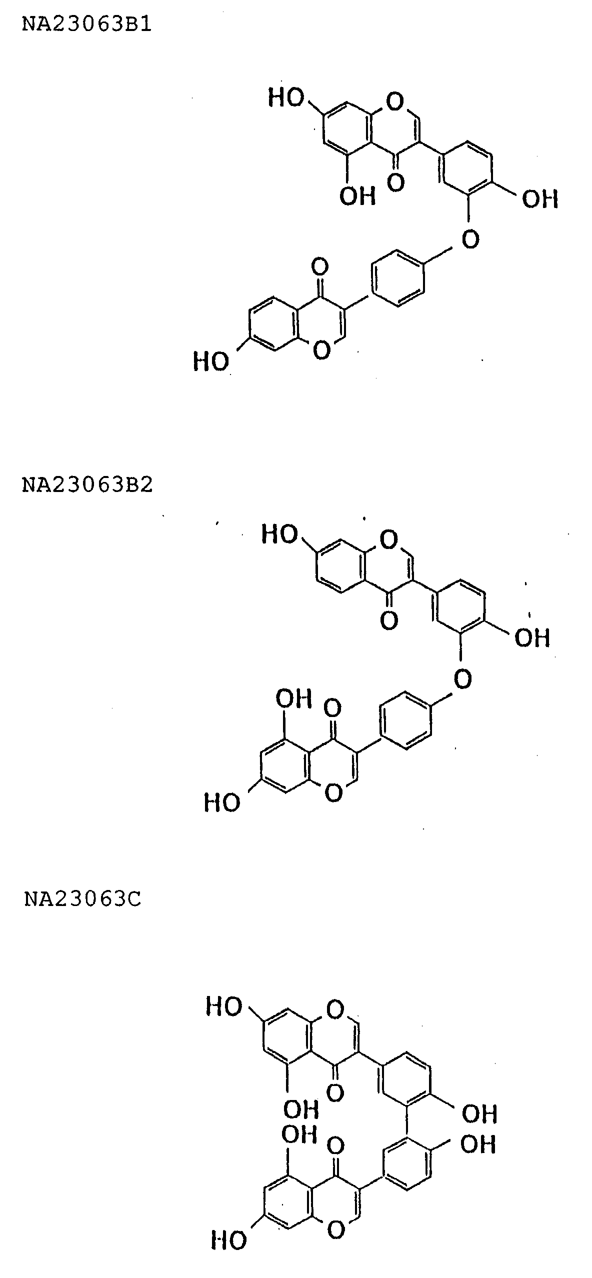

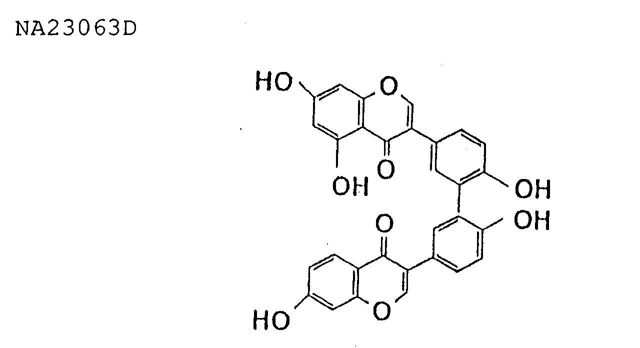

As one typical strain capable of producing NA23063A, B1, B2, C and D, mentioned is Streptomyces sp. NA23063 which was isolated from soil. This strain was deposited in the International Depositary Authority, the National Institute of Bioscience and Human-Technology Agency of Industrial Science and Technology of Japan, according to the Budapest Treaty (P-14685, FERM BP-5490).

### BRIEF DESCRIPTION OF THE DRAWINGS

Fig. 1 shows the pattern of UV absorption spectrum of NA23063A as measured in methanol.

Fig. 2 shows the pattern of IR absorption spectrum of NA23063A as measured in potassium bromide tablet.

Fig. 3 shows the pattern of UV absorption spectrum of NA23063B1 as measured in methanol.

Fig. 4 shows the pattern of IR absorption spectrum of NA23063B1 as measured in potassium bromide tablet.

Fig. 5 shows the pattern of UV absorption spectrum of NA23063B2 as measured in methanol.

Fig. 6 shows the pattern of IR absorption spectrum of NA23063B2 as measured in potassium bromide tablet.

Fig. 7 shows the pattern of UV absorption spectrum of NA23063C as measured in methanol.

Fig. 8 shows the pattern of IR absorption spectrum of NA23063C as measured in potassium bromide tablet.

Fig. 9 shows the pattern of UV absorption spectrum of NA23063D as measured in methanol.

Fig. 10 shows the pattern of IR absorption spectrum of NA23063D as measured in potassium bromide tablet.

### BEST MODES OF CARRYING OUT THE INVENTION

The C₁₋₄ lower alkyl group referred to herein includes, for example, methyl, ethyl, propyl, i-propyl, n-butyl and t-butyl groups. Preferred are methyl and ethyl groups. The group -(CH₂)nCOOH indicates, for example, methylcarboxylic acid, ethylcarboxylic acid, propylcarboxylic acid, i-propylcarboxylic acid, n-butylcarboxylic acid and t-butylcarboxylic acid. Preferred are methylcarboxylic acid and ethylcarboxylic acid. The group -(CH₂)ₙNYY includes, for example, methylamine, ethylamine, propylamine, methylaminomethyl, methylaminoethyl, methylaminodimethyl, methylaminodiethyl, ethylaminomethyl, ethylaminoethyl, ethylaminodimethyl, propylaminomethyl, propylaminoethyl and propylaminodimethyl groups. Preferred are methylaminomethyl, methylaminodimethyl and methylaminodiethyl groups. The C₁₋₄ lower alkylcarbonyl group includes, for example, methylcarbonyl, ethylcarbonyl, propylcarbonyl, i-propylcarbonyl, n-butylcarbonyl and t-butylcarbonyl groups. Preferred are methylcarbonyl and ethylcarbonyl groups. The group -CO(CH₂)ₙCOOH includes, for example, carboxymethylcarbonyl, 2-carboxyethylcarbonyl, 3-carboxypropylcarbonyl and 4-carboxybutylcarbonyl groups. Preferred are carboxymethylcarbonyl and 2-carboxyethylcarbonyl groups. The group -CO(CH₂)ₙNYY includes, for example, those of the above-mentioned - (CH₂)ₙNYY to which is bonded a carbonyl group. The compounds with any of those OR groups are expected to be well delivered in bodies. In the compounds having two or more hydroxyl groups, it is preferred that one hydroxyl group is substituted to be OR while the others are not substituted.

As the pharmaceutically acceptable salts referred to herein, mentioned are alkali salts of the compounds where the functional group has a carboxyl group. Preferred are sodium, potassium or calcium salts of the compounds. The compounds where the functional group has an amino group form salts with acids such as hydrochloric acid, sulfuric acid and phosphoric acid.

The compound of formula (1) where R₁, R₃, R₄ and R₆ are hydroxyl groups, R₂ and R₅ are hydrogen atoms and X is a direct bond is known as Kudzuisoflavone A (its structural formula is mentioned below), which is said to be isolated from an yeast Puerarialobata, but its pharmaceutical effects are not disclosed (see Z. Naturforsch, 47C, pp. 177-182, 1992).

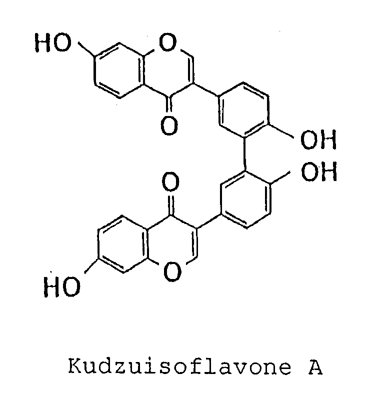

This compound is structurally similar to NA23063A, B1, B2, C and D of the invention, and is expected to have the effect of inhibiting phosphodiesterases like the compounds of the invention.

The microorganisms capable of producing the physiologically active substances NA23063A, B1, B2, C and D of the invention, which have the effect of inhibiting phosphodiesterases, belong to the genus Streptomyces. A strain NA23063 isolated by the present inventors (FERM P-14685 - this was deposited in the International Depositary Authority according to the Budapest Treaty as FERM BP-5490) is one example that is most effectively used in the invention.

The novel physiologically active substances NA23063A, B1, B2, C and D can be obtained by cultivating microorganisms belonging to the genus Streptomyces and capable of producing NA23063A, B1, B2, C and D to make them produce the compounds NA23063A, B1, B2, C and D, followed by collecting those physiologically active substances NA23063A, B1, B2, C and D thus accumulated in the culture.

One typical strain capable of producing the physiologically active substances NA23063A, B1, B2, C and D has the following morphological and physiological properties.

### 1. Morphological Properties:

After having been cultivated at 27°C for 2 weeks, the cells were found to have simple branched aerial mycelia, of which the tips were looped or spiral, with no verticillate branches formed. Neither sporangia nor zoospores were found to be formed. The surfaces of the spores formed were smooth or rough, the spores were cylindrical and were 0.7 to 0.9 x 1.0 to 1.4 µm in size. Ten or more spores formed were chained in series.

### 2. Growth in Different Media:

The growth of the cells in different media at 27°C for 2 weeks is shown in Table 1 below.

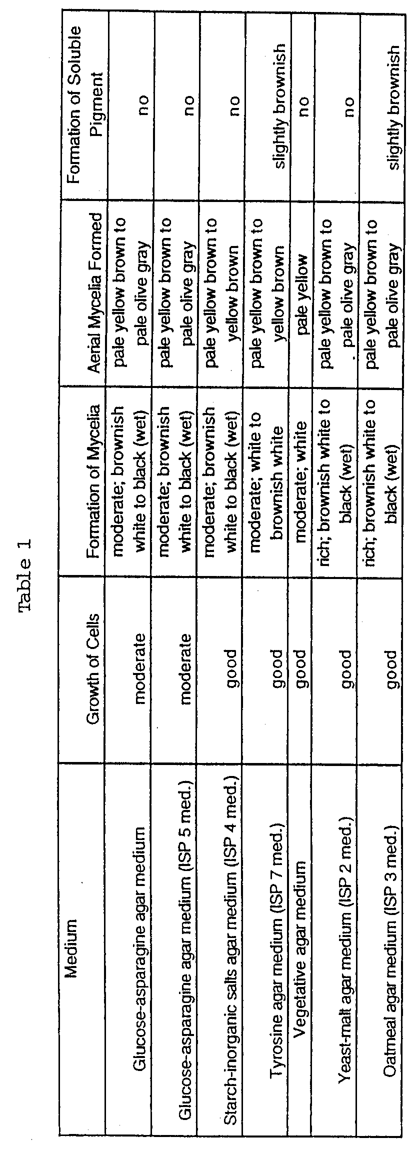

### 3. Physiological Properties:

1. Growth temperature range: 27 to 37°C
2. Reduction of nitrates: negative
3. Liquefaction of gelatin (in glucose-peptone-gelatin medium, 20°C): negative
4. Hydrolysis of starch (starch-inorganic salts agar medium): positive
5. Coagulation of skim milk: negative
6. Peptonization of skim milk: negative
7. Formation of melanoid pigment: negative

### 4. Utilization of Carbon Sources (in Pridham and Gottlieb agar medium):

- L-arabinose:: +
- D-xylose:: +
- D-glucose:: +
- D-fructose:: +
- Sucrose:: +
- Inositol:: +
- L-rhamnose:: +/-
- Raffinose:: +
- D-mannitol:: +

### 5. Diaminopimelic Acid in Cell Walls:

LL-diaminopimelic acid was found.

### 6. Type of Menaquinone:

MK9 (H 8) and MK9(H 6) were found.

As above, the cell walls of this strain contain LL-diaminopimelic acid and menaquinones of MK-9 (H 8) and MK-9 (H 6). According to the method of International Streptomyces Project (ISP), the morphology of the mycelia formed around the spores of the strain belongs to the section Spairales. The surfaces of the spores are smooth or rough, and the color of the matured aerial mycelia is in gray color series. With further maturation, the aerial mycelia become black (wet). No melanoid pigment is formed by the strain, but the culture of the cells is slightly yellowish. The color of the aerial mycelia is pale yellow brown to pale olive. As the carbon sources, the strain utilizes L-arabinose, D-glucose, D-fructose, D-xylose, inositol, sucrose, D-mannitol and raffinose, but little rhamnose.

Based on these results, this NA 23063 strain was found to belong to the genus Streptomyces from Bergey's Manual of Determinative Bacteriology, 8th Ed., 1974 (edited by R.E. Buffanan and N.E. Gibbons). Accordingly, this strain was named Streptomyces sp. NA 23063.

This strain was deposited in the International Depositary Authority, the National Institute of Bioscience and Human-Technology Agency of Industrial Science and Technology of Japan, under the accession number FERM P-14685 and FERM BP-5490 according to the Budapest Treaty.

Like those of other strains of the genus Streptomyces, the properties of this strain easily vary, and this strain can be easily mutated by some artificial means of using, for example, ultraviolet rays, X-rays or chemicals. All mutants of this strain can be used in the invention so far as they have the ability to produce the physiologically active substances NA23063A, B1, B2, C and D of the invention.

To produce NA23063A, B1, B2, C and D in accordance with the invention, the strain is aerobically cultivated in a medium comprising the nutrients capable of being utilized by the strain. The nutrient sources may be any conventional ones which have heretofore been used in the cultivation of actinomycetes. For example, the carbon source may be any of glucose, fructose, glycerin, sucrose, dextrin, galactose and organic acids, which can be used either singly or as combined.

The inorganic and organic nitrogen source may be any of ammonium chloride, ammonium sulfate, urea, ammonium nitrate, sodium nitrate, peptone, meat extract, yeast extract, dry yeast, corn steep liquor, soybean meal, cotton seed refuse, Casamino acid, bactosoiton, soluble vegetable protein and oatmeal, which can be used either singly or as combined.

If desired, any other inorganic salts, such as sodium chloride, calcium carbonate, magnesium sulfate, copper sulfate, iron sulfate, zinc sulfate, manganese chloride and phosphates, may be added to the medium. In addition, any other organic substances, such as amino acids, vitamins and nucleic acids, and even other inorganic substances may also be added to the medium. To cultivate the cells, preferred is liquid culture, and submerged culture is the best. It is desirable that the cells are cultivated at a temperature falling between 20 and 45°C, and in a slightly acidic or slightly alkaline pH condition.

After having been cultivated in liquid culture generally for from 3 to 5 days, the cells produce NA23063A, B1, B2, C and D in the culture. When the amount of the active substances produced by the cells has reached the maximum, the cultivation of the cells is stopped. Then, the cells are separated from the medium, and the intended products are isolated and purified from the cells. To isolate and purify the active substances from the cells, employable is any known method for isolating the microbial metabolites from cultivated cells.

Precisely, the culture is filtered in an ordinary manner to separate the cells from the filtrate. The thus-obtained cells are suspended in acetone and then filtered to obtain an acetone extract. This extract is concentrated under reduced pressure to remove acetone, then re-dissolved in an ordinary organic solvent that is not miscible with water, and concentrated under reduced pressure to obtain an oily component comprising NA23063A, B1, B2, C and D.

This oily component is then purified in any known method for purification of fat-soluble substances, for example, through silica gel column chromatography and/or recrystallization. One preferred example for the purification is silica gel column chromatography using n-hexane/acetone or n-hexane/ethyl acetate as the eluent. The resulting active fraction is further subjected to ODS column chromatography (mobile phase: water/methanol) to obtain NA23063A, NA23063B1, NA23063B2, NA23063C and NA23063D.

The thus-obtained, physiologically active substances NA23063A, B1, B2, C and D have the following physicochemical properties.

### Physiologically active substance NA23063A:

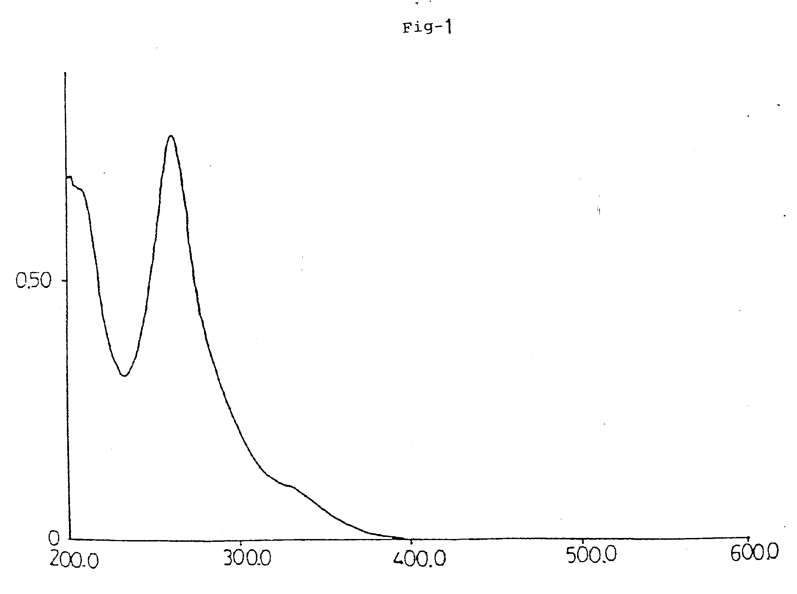

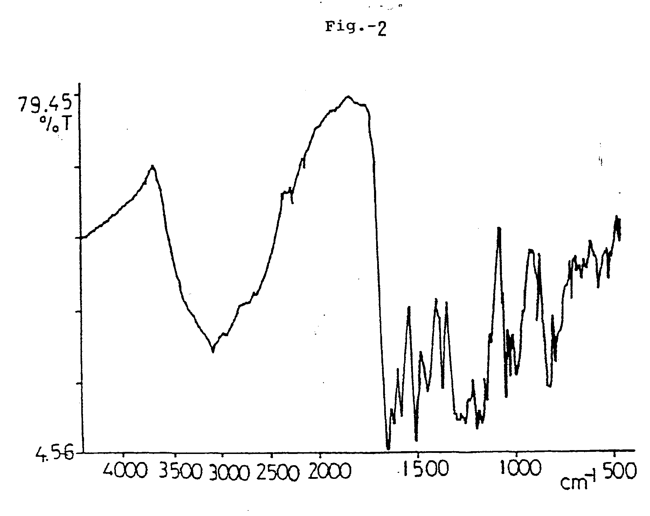

1) Appearance: yellow powder
2) Molecular weight: 538
3) Molecular formula: C₃₀H₁₈O₁₀
4) Solubility: soluble in lower alcohols, acetone and ethyl acetate, but insoluble in hexane, petroleum ether and water.
5) Rf in silica gel thin-layer chromatography using a developer of hexane/acetone (1/1): 0.38
6) UV absorption spectrum: Fig. 1
7) IR absorption spectrum (in KBr tablet): Fig. 2
8) ¹H-NMR spectrum (300 MHz, in dimethylsulfoxide-d₆):
   12.87 (1H, s), 12.86 (1H, s), 8.36 (1H, s), 8.34 (1H, s), 7.50 (2H, d, J = 8.8 Hz), 7.30 (1H, dd, J = 8.7, 2.1 Hz), 7.05 (1H, d, J = 8.7 Hz), 6.93 (2H, d, J = 8.8 Hz), 6.37 (1H, d, J = 2.1 Hz), 6.35 (1H, d, J = 2.1 Hz), 6.21 (1H, d, J = 2.1 Hz), 6.20 (1H, d, J = 2.1 Hz) ppm
9) ¹³C-NMR spectrum (75 MHz, in dimethylsulfoxide-d₆):
   180.0 (s), 179.9 (s), 164.6 (s), 164.5 (s), 162.0 (s), 157.9 (s), 157.6 (s), 157.5 (s), 154.3 (d), 149.5 (s), 141.7 (s), 130.3 (d), 126.3 (d), 124.4 (s), 122.9 (s), 122.3 (d), 121.9 (s), 121.4 (s), 117.1 (d), 115.6 (d), 104.4 (s), 99.1 (d), 93.7 (d) ppm

### Physiologically active substance NA23063B1:

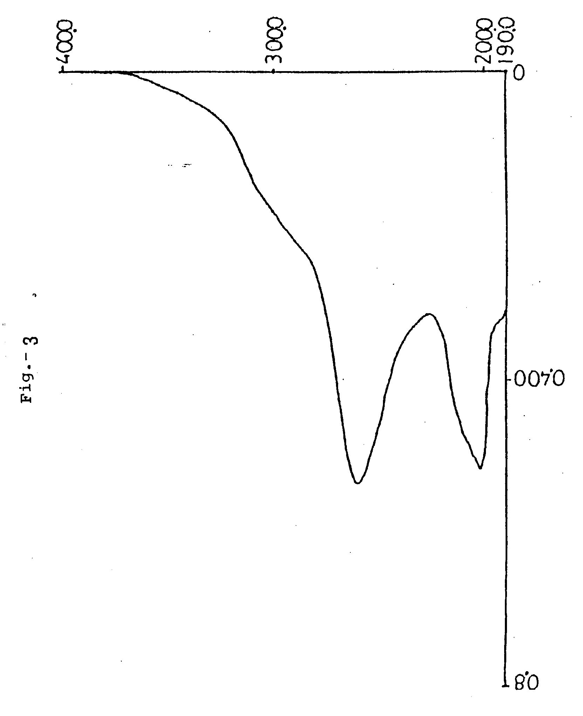

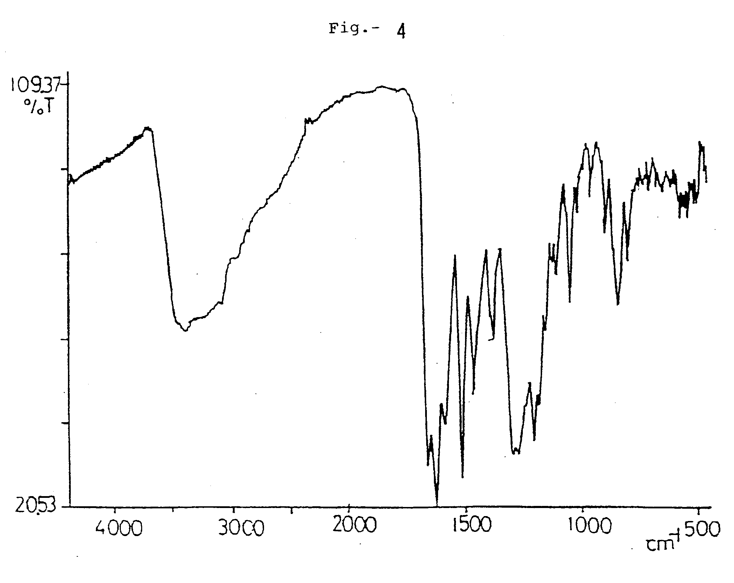

1) Appearance: yellow powder
2) Molecular weight: 522
3) Molecular formula: C₃₀H₁₈O₉
4) Solubility: soluble in lower alcohols, acetone, ethyl acetate and dimethylsulfoxide, but insoluble in hexane, petroleum ether and water.
5) Rf in silica gel thin-layer chromatography using a developer of hexane/acetone (1/1): 0.36
6) UV absorption spectrum: Fig. 3
7) IR absorption spectrum (in KBr tablet): Fig. 4
8) ¹H-NMR spectrum (300 MHz, in dimethylsulfoxide-d₆):
   12.9 (1H, s), 8.38 (1H, s), 8.33 (1H, s), 7.96 (1H, d, J = 2.2 Hz), 7.50 (2H, d, J = 8.8 Hz), 7.31 (1H, d, J = 2.1 Hz), 7.30 (1H, dd, J = 8.9, 2.1 Hz), 7.04 (1H, d, J = 8.9 Hz), 6.94 (2H, d, J = 8.8 Hz), 6.91 (1H, dd, J = 8.8, 2.2 Hz), 6.86 (1H, d, J = 2.2 Hz), 6.36 (1H, d, J = 2.1 Hz), 6.20 (1H, d, J = 2.1 Hz) ppm
9) ¹³C-NMR spectrum (75 MHz, in dimethylsulfoxide-d₆):
   180.0 (s), 174.5 (s), 164.5 (s), 162.7 (s), 162.0 (s), 157.7 (s), 157.6 (s), 157.5 (s), 154.3 (d), 153.2 (d), 149.5 (s), 141.7 (s), 130.2 (d), 127.3 (d), 126.1 (d), 124.3 (s), 123.1 (s), 122.7 (d), 122.5 (s), 121.9 (s), 117.0 (s), 116.5 (d), 115.6 (d), 115.2 (d), 104.4 (s), 102.5 (d), 99.1 (d), 93.7 (d) ppm
10) Color reaction: positive to iodine.

### Physiologically active substance NA23063B2:

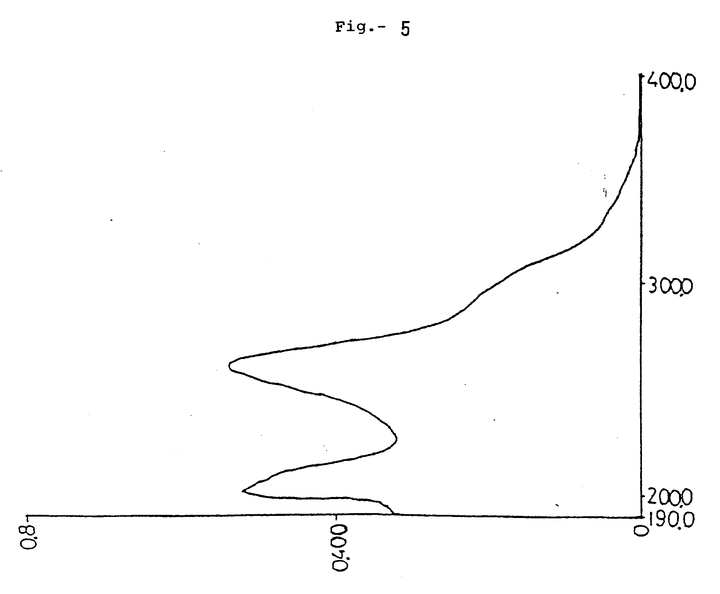

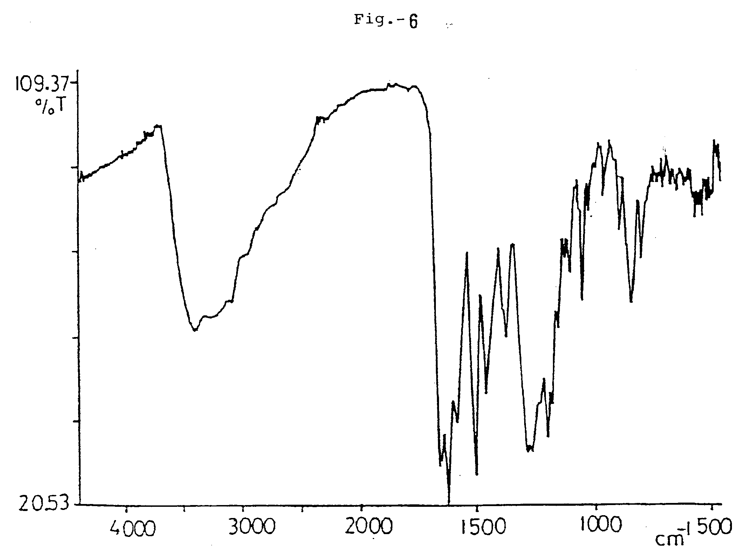

1) Appearance: yellow powder
2) Molecular weight: 522
3) Molecular formula: C₃₀H₁₈O₉
4) Solubility: soluble in lower alcohols, acetone, ethyl acetate and dimethylsulfoxide, but insoluble in hexane, petroleum ether and water.
5) Rf in silica gel thin-layer chromatography using a developer of hexane/acetone (1/1): 0.36
6) UV absorption spectrum: Fig. 5
7) IR absorption spectrum (in KBr tablet): Fig. 6
8) ¹H-NMR spectrum (300 MHz, in dimethylsulfoxide-d₆):
   12.9 (1H, s), 8.36 (1H, s), 8.36 (1H, s), 7.94 (1H, d, J = 8.8 Hz), 7.50 (2H, d, J = 8.8 Hz), 7.31 (1H, d, J = 2.1 Hz), 7.30 (1H, dd, J = 8.2, 2.1 Hz), 6.92 (2H, d, J = 8.8 Hz), 6.91 (1H, dd, J = 8.8, 2.1 Hz), 6.84 (1H, d, J = 2.2 Hz), 6.38 (1H, d, J = 2.1 Hz), 6.22 (1H, d, J = 2.1 Hz) ppm
9) ¹³C-NMR spectrum (75 MHz, in dimethylsulfoxide-d₆):
   180.0 (s), 174.5 (s), 164.5 (s), 162.7 (s), 162.0 (s), 157.9 (s), 157.5 (s), 157.4 (s), 154.3 (d), 153.2 (d), 149.2 (s), 141.7 (s), 130.3 (d), 127.3 (d), 126.2 (d), 125.7 (d), 123.6 (s), 122.8 (s), 122.3 (d), 121.4 (s), 117.1 (s), 116.5 (d), 115.7 (d), 115.2 (d), 104.4 (s), 102.5 (d), 99.1 (d), 93.7 (d) ppm
10) Color reaction: positive to iodine.

### Physiologically active substance NA23063C:

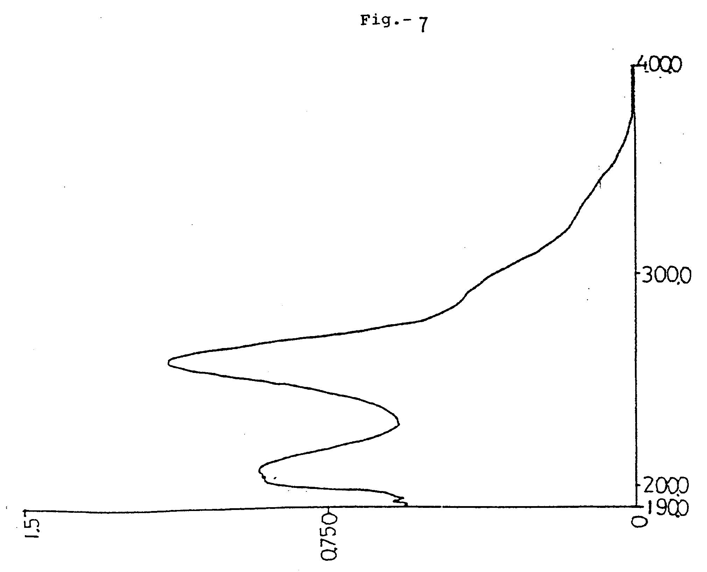

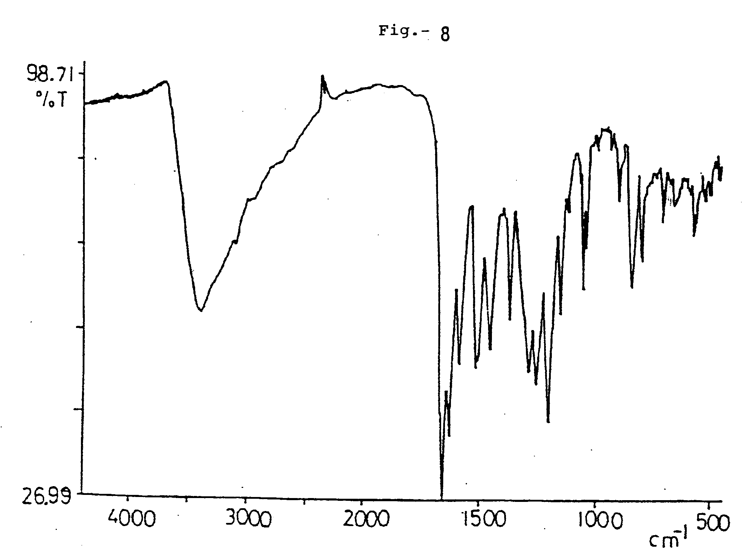

1) Appearance: yellow powder
2) Molecular weight: 538
3) Molecular formula: C₃₀H₁₈O₁₀
4) Solubility: soluble in lower alcohols, acetone, ethyl acetate and dimethylsulfoxide, but insoluble in hexane, petroleum ether and water.
5) Rf in silica gel thin-layer chromatography using a developer of hexane/acetone (1/1): 0.33
6) UV absorption spectrum: Fig. 7
7) IR absorption spectrum (in KBr tablet): Fig. 8
8) ¹H-NMR spectrum (300 MHz, in dimethylsulfoxide-d₆):
   13.0 (2H, s), 8.35 (2H, s), 7.41 (2H, d, J = 2.2 Hz), 7.37 (2H, dd, J = 8.3, 2.2 Hz), 6.94 (2H, d, J = 8.3 Hz), 6.37 (2H, d, J = 1.9 Hz), 6.21 (2H, d, J = 1.9 Hz) ppm
9) ¹³C-NMR spectrum (75 MHz, in dimethylsulfoxide-d₆):
   180.0 (s), 174.5 (s), 164.5 (s), 162.7 (s), 162.0 (s), 157.9 (s), 157.5 (s), 157.4 (s), 154.3 (d), 153.2 (d), 149.2 (s), 141.7 (s), 130.3 (d), 127.3 (d), 126.2 (s), 125.7 (d), 123.6 (s), 122.8 (s), 122.3 (d), 121.4 (s), 117.1 (s), 116.5 (d), 115.7 (d), 115.2 (d), 104.4 (s), 102.5 (d), 99.1 (d), 93.7 (d) ppm
10) Color reaction: positive to iodine.

### Physiologically active substance NA23063D:

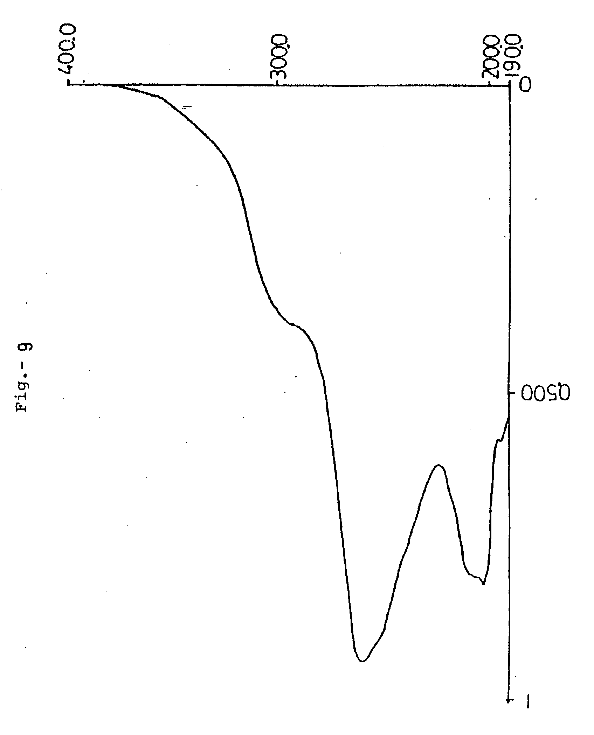

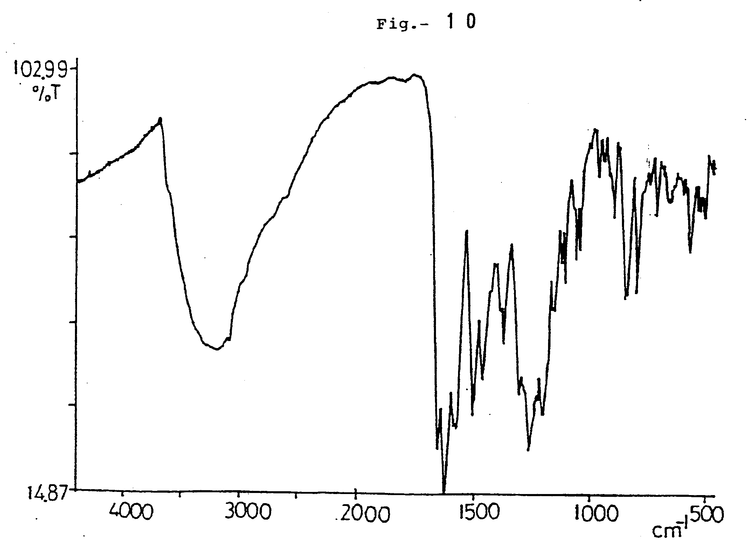

1) Appearance: yellow powder
2) Molecular weight: 522
3) Molecular formula: C₃₀H₁₈O₉
4) Solubility: soluble in lower alcohols, acetone, ethyl acetate and dimethylsulfoxide, but insoluble in hexane, petroleum ether and water.
5) Rf in silica gel thin-layer chromatography using a developer of hexane/acetone (1/1): 0.26
6) UV absorption spectrum: Fig. 9
7) IR absorption spectrum (in KBr tablet): Fig. 10
8) ¹H-NMR spectrum (300 MHz, in dimethylsulfoxide-d₆):
   13.0 (1H, s), 8.35 (1H, s), 8.32 (1H, s), 7.95 (1H, d, J = 8.8 Hz), 7.42 (2H, d, J = 2.3 Hz), 7.37 (1H, dd, J = 8.3, 2.3 Hz), 7.36 (1H, dd, J = 8.3, 2.3 Hz), 6.93 (1H, d, J = 8.3 Hz), 6.92 (1H, d, J = 8.3 Hz), 6.91 (1H, dd, J = 8.8, 2.2 Hz), 6.84 (1H, d, J = 2.2 Hz), 6.32 (1H, d, J = 2.1 Hz), 6.21 (1H, d, J = 2.1 Hz) ppm
9) ¹³C-NMR spectrum (75 MHz, in dimethylsulfoxide-d₆):
   180.3 (s), 174.8 (s), 164.6 (s), 162.7 (s), 162.0 (s), 157.6 (s), 157.5 (s), 155.8 (s), 155.5 (s), 154.0 (d), 152.8 (d), 131.8 (d), 131.7 (d), 128.9 (d), 127.3 (d), 126.2 (s), 126.0 (d), 123.6 (s), 122.4 (s), 122.1 (d), 120.7 (s), 116.6 (s), 116.0 (d), 115.9 (d), 115.2 (d), 104.4 (s), 102.1 (d), 99.0 (d), 93.7 (d) ppm
10) Color reaction: positive to iodine.

Compounds of formula (1) of the invention can be produced from NA23063A, B1, B2, C and D, which are obtained from the strain of Streptomyces sp. NA 23063, and from Kudzuisoflavone A.

Precisely, where the hydroxyl group in formula (1) is desired to be substituted with R of an alkyl group, such as a C₁₋₄ lower alkyl group, -(CH₂)ₙCOOH or -(CH₂)ₙNYY, the compound is dissolved in an aprotic polar solvent (e.g., acetone, dimethylsulfoxide, dimethylformamide), and reacted with the corresponding alkyl, ω-alkoxycarbonylalkyl or ω-dialkylaminoalkyl halide at room temperature or under heat in the presence of a base (e.g., potassium carbonate, tertiary amine) to obtain the intended alkylated derivative.

Where the hydroxyl group in formula (1) is desired to be substituted with R of an acyl group, such as a C₁₋₄ lower alkylcarbonyl group, -CO(CH₂)ₙCOOH or -CO(CH₂)ₙNYY, the compound is dissolved in a solvent of pyridine, and reacted with the corresponding acid chloride or anhydride at room temperature or under heat to obtain the intended acylated derivative.

Where the starting compound to be alkylated or acylated has from 2 to 6 hydroxyl groups, one or more of those hydroxyl groups may be partly alkylated or acylated by controlling the number of mols of the reactant, thereby obtaining partially alkylated or acylated derivatives. It is also possible to use an excessive amount of the reactant to substitute all those hydroxyl groups to give completely alkylated or acylated derivatives. The position of the substituent as introduced as a result of the reaction can be identified through NMR or the like.

Where the compounds of the invention are used in medicines, any and every known technique may apply to their formulation and administration. For example, to administer the compounds, employable is any of injection, oral administration and rectal administration. Regarding the formulation of the compounds, they can be formed into any of injections, powders, granules, tablets and suppositories.

To formulate the compounds, NA23063 analogues into medicines, optionally employed are various additives not having any negative influence on those compounds, for example, carriers and other aids, such as stabilizers, preservatives, pain relievers, emulsifiers, etc.

The content of NA23063 analogues in pharmaceutical compositions may vary in a broad range, depending on the form of the compositions. In general, however, the pharmaceutical compositions may contain from 0.01 to 100 % by weight, preferably from 0.1 to 70 % by weight, of NA23063 analogues, with the balance being ordinary, pharmaceutically-cceptable carriers and aids.

The dose of NA23063 analogues varies depending on the disease condition and other factors, but may be approximately from 0.01 to 800 mg/adult/day. When the compounds are continuously administered, it is desirable that the dose a day is lowered.

The following Test Examples are to demonstrate the phosphodiesterases inhibiting effect of NA23063A, B1, B2, C and D.

### Test Example 1:

### Preparation of Bovine Phosphodiesterase:

Fifity g of the respiratory smooth muscle of a sacrificed cow was cut into pieces with scissors and a knife, then suspended in 2 mM EDTA-containing 20 mM Tris buffer (pH 7.4) of 5 times by volume the pieces, and homogenized with a polytron homogenizer to prepare a crude phosphodiesterase liquid. This was centrifuged at 10,000 x g for 20 minutes to collect the supernatant, which is a soluble, crude phosphodiesterase liquid. This was put into a Q-Sepharose fast-flow column (100 ml), washed with a buffer, and then eluted with sodium acetate having a gradient concentration of from 50 mM to 1 M. The phosphodiesterase-containing active fraction eluted with 0.7 M sodium acetate was collected.

### Determination of Phosphodiesterase Activity:

To determine its phosphodiesterase activity, the enzyme was reacted with a substrate, 3',5'-cyclic adenosine monophosphate (cAMP, manufactured by Sigma Co. in US). After the reaction, the remaining cAMP was measured through high-performance liquid chromatography.

0.02 mg/ml of cAMP, 2.5 mM dithiothreitol, 6 mM magnesium chloride, 50 mM Tris buffer (pH 8.0), 1 µl of the phosphodiesterase liquid prepared above, and a varying amount of the compound of the invention were put into plastic tubes having a volume of 0.3 ml, to which was added water to have a final volume of 200 µl. The mixture was stirred, and incubated at 37°C for 60 minutes. 20 µl of an aqueous solution of 200 mM tetraethylenediamine was added to each tube to stop the reaction. 20 µl of the resulting reaction mixture was subjected to high-performance liquid chromatography (ODS column of 3.9 mm⌀ x 150 mm, manufactured by Waters Co. in US), through which the remaining cAMP was measured. From the data, obtained was the activity of the compound of the invention of inhibiting the enzyme phosphodiesterase, as in Table 2.

**Table 2**

| Phosphodiesterase Inhibiting Activity | | | | | |
|---|---|---|---|---|---|
| Concentration (µg/ml) | Inhibiting Activity (%) | | | | |
| | NA23063A | NA23063B1 | NA23063B2 | NA23063C | NA23063D |
| 6.25 | 79.5 | 64.9 | 64.9 | 83.8 | 61.0 |
| 3.13 | 60.5 | 56.3 | 56.3 | 77.6 | 44.8 |
| 1.56 | 57.8 | 38.2 | 38.2 | 66.5 | 34.6 |
| 0.78 | 43.5 | 28.1 | 28.1 | 57.7 | 30.7 |
| 0.39 | - | 17.5 | 17.5 | 26.4 | 18.9 |

As in the above, NA23063A, B1, B2, C and D all have the high effect of inhibiting phosphodiesterase, and their IC 50 values are 2.1 x 10⁻⁶ M, 4.9 x 10⁻⁶ M, 4.9 x 10⁻⁶ M, 1.3 x 10⁻⁶ M, and 7.9 x 10⁻⁶ M, respectively.

### Test Example 2:

### Effect of Inhibiting Contraction of Bronchial Smooth Muscle:

The tracheae were taken out of male Hartley guinea pigs having a body weight of from 400 to 500 g. These were cut off at the cartilage opposite to the smooth muscle, and sliced into small pieces having a width of 1 mm. Five of these pieces were bound with silk thread into one bundle, of which the both ends were ligated with machine cotton. This was then suspended in an organ bath with applying thereto a tension of 0.5 g. The temperature of the bath was 37°C, and the bath contained a nutrient liquid of Krebs-Hanseleit buffer (118 mM NaCl, 4.6 mM KCl, 1.1 mM MgSO₄·7H₂O, 1.8 mM CaCl₂·2H₂O, 24.9 mM NaHCO₃, 1.0 mM KH₂PO₄·2H₂O, 11.1 mM glucose). To the nutrient liquid, added was 5 µM indomethacin. The bath was well aerated with a mixed gas of 95 % O₂ + 5 % CO₂. The suspended tissue was incubated in the organ bath for about 2 hours for habituation in the external environment. Then, this was contracted with 10 µM histamine. After the contraction was stabilized, NA23063D was added to the bath to have a concentration of 0.1, 0.3, 1.0, 3.0, 10 and 30 µg/ml. The reaction in the bath comprising the varying amount of NA23063D was continued until the relaxation of the tissue became stabilized. When no relaxation was admitted in 10 minutes after the addition of the chemical, NA23063D, the concentration of the chemical was increased to the next stage. The compound, NA23063D was found soluble in dimethylsulfoxide (DMSO). Therefore, in order to examine the influence of DMSO on the present test system, DMSO of the same concentration as that the compound NA23063D was added to the organ bath in place of the compound NA23063D to prepare control groups. After the entire reaction, 0.2 mM papaverine was added to each bath to determine the maximum relaxation of the tissue.

The highest level of contraction with 10 µM histamine was referred to as an index of 100 %, while the highest level of relaxation with 0.2 mµ papaverine was as 0 %. Relative to those indices, 10 µg/ml of NA23063D produced the relaxation of the bronchial smooth muscle of 61 %.

### Test Example 3:

### Activity of Inhibiting Activation of Oxyphiles:

### (A) Preparation of Oxyphiles:

One mg of polymyxin B (manufactured by Sigma Co.) was intra-abdominally administered to Hartley guinea pigs (6-week age). The chemical was administered to the animals once a week, for 6 to 8 weeks. After the final administration of the chemical polymyxin B, 50 ml of PBS (phosphate-buffered saline) was injected into the abdomen of each guinea pig, and the abdominal cells were collected. These were centrifuged at 800 rpm for 5 minutes to collect the precipitate of abdominal infiltrated cells. The precipitated cells were suspended in 1 ml of 40 % Ficol solution (40 % Ficol (manufactured by Pharmacia Co.) in HBSS (Hanks' conditioned salt solution)), and stratified over a discontinuous density gradient of Ficol (40 to 90 %). This was centrifuged at 1500 rpm for 40 minutes to collect the oxyphile fraction. This operation generally produced oxyphiles having a purity of 99 % or higher. The cells were washed two times with an RPMI1640 medium (0.2 % BSA (bovine serum albumin), manufactured by Boehringer Mannheim Co.), then suspended to have a predetermined cell concentration, and used in the intended experiments. (B) Effect of Inactivating Oxyphiles (Effect of inhibiting the activity of guinea pig oxyphiles to release active oxygen):

The oxyphiles obtained in the Ficol density gradient method in (A) (these had a purity of 99 % or more) were suspended in a buffer (0.136 M NaCl, 2.7 mM KCl, 1.8 mM CaCl₂, 1.0 mM MgCl₂, 5.5 mM D-glucose, 11.9 mM NaHCO₃, 5 mM HEPES, 0.36 mM NaH₂PO₄, pH 7.2) in an amount of 4 x 10⁵ cells.

2 µl of each chemical to be tested and 180 µl of the cell suspension were added to each well of a 96-well multiwell plate (white plate for chemiluminometry) which had been previously kept at 37°C, and kept therein at 37°C for 10 minutes. After thus kept, 20 µl of PAF (platelet activating factor) (x -10⁻⁶ M) and 20 µl of a luminol solution were added thereto, and immediately the intensity of chemiluminescence of the system was measured, using Labosystems (manufactured by Luminoskan Co.). The data of chemiluminescence for 5 minutes were obtained, from which was calculated the 50 % inhibition concentration of the compound tested (IC₅₀). The data of IC₅₀ are shown in Table 3.

**Table 3**

| Activity of Inhibiting Activation of Oxyphiles | |
|---|---|
| Compound | IC₅₀ (x 10⁻⁶ M) |
| NA23063A | 0.1 |
| NA23063C | 0.32 |
| NA23063D | 1.1 |

### Test Example 4:

### Effect in Asthmatic Animal Models:

### (A) Preparation of Immediate Allergic Respiratory Stenosis Models:

An antigen ovalbumin (OA) was administered to male Brown-Norway (BN) rats to prepare actively-sensitized animal models. To sensitize the animals, 1 ml of the antigen solution (100 mg/ml of Al(OH)₃ and 1 mg/ml of OA in aqueous 0.9 % NaCl solution) was subcutaneously administered to the back of each rat. On the other hand, inactivated cells of Bordetella pertussis were suspended in an aqueous solution of 0.9 % NaCl to have a concentration of 1.0 x 10¹⁰ cells/ml. 0.25 ml of the cell suspension was intra-abdominally administered to each rat along with the antigen sensitization. The administration was repeated three times in all, on the 1st, 7th and 14th days. During the period of from the 21th day to the 28th day after the start of the sensitization, OA in each animal was challenged. Thus were prepared experimental, immediate allergic respiratory stenosis models.

### (B) Effect of Inhibiting Immediate Allergic Respirator Stenosis:

Those actively-sensitized BN rats were anesthetized with sodium pentobarbiturate (50 mg/kg, i.p.), into which was inserted a polyvinyl chloride tube cannula through the respiratory tract. Each rat was purged with air through the cannula, and the respiratory tract resistance was monitored. The purging with air was effected at 60 strokes/min in an amount of 100 ml/kg. In addition, an additional cannula was inserted into the left common carotid artery of each rat, through which the blood pressure and the heart beat of the rat being tested were monitored. Moreover, a still further cannula was inserted into the right common carotid artery of each rat, through which 30 mg/kg of NA23063D and 5 mg of the antigen (OA) were administered. On the basis of the time (0, zero) at which the antigen was challenged, the variation (increase or decrease) in the respiratory tract resistance of each rat was recorded at regular intervals before and after the administration of the antigen.

Based on the maximum respiratory tract resistance (0 %) of the control animals (to which no test chemical was administered) after the administration of the antigen, the decrease in the respiratory tract resistance of the tested animals (to which the test chemical was administered) was obtained in terms of % inhibition.

As a result, the intravenous administration of 30 mg/kg of NA23063D to the rat models produced 54 % inhibition.

### EXAMPLES

Examples of the invention are mentioned below, which are to exemplify the invention but are not to restrict the invention. The invention shall encompass various changes and modifications of those Examples.

### Production Example:

A plurality of 500 ml-Erlenmeyer flasks for rotary shakers each were charged with 100 ml of a medium (pH 7.2) comprising 0.5 % glycerin, 2.0 % starch, 0.5 % soybean meal, 0.5 % yeast extract, 0.05 % dipotassium phosphate, 0.05 % magnesium sulfate and 0.2 % potassium carbonate, and sterilized in an autoclave at 120°C for 20 minutes. One platinum loop of cells of a strain NA 23063 (P-14685, deposited in the National Institute of Bioscience and Human-Technology Agency of Industrial Science and Technology of Japan) was inoculated into the medium, and shaken at 27°C at 220 rpm for 2 days. Apart from those, a plurality of 500 ml-Erlenmeyer flasks for rotary shakers each were charged with 100 ml of a medium (pH 7.2) comprising 0.5 % glycerin, 2.0 % starch, 0.5 % soybean meal, 0.5 % yeast extract, 0.05 % dipotassium phosphate, 0.05 % magnesium sulfate and 0.2 % potassium carbonate, and sterilized in an autoclave at 120°C for 20 minutes, and one ml of the culture prepared above was transplanted in the medium, and shaken at 27°C at 220 rpm for 2 days to prepare a seed culture.

A 200 liter-incubator for submerged culture was charged with 120 liters of a medium (pH 7.0) comprising 2.0 % glycerin, 2.0 % dextrin, 2.0 % soybean meal, 0.5 % corn steep liquor, 0.05 % dipotassium phosphate, 0.05 % magnesium sulfate and 0.2 % potassium carbonate, and sterilized with steam at 120°C for 20 minutes. The seed culture (1.2 liters) prepared above was transplanted into this medium, and incubated with stirring at 27°C, at 270 rpm, and at an aeration rate of 2/3 volume/min, for 4 days.

The resulting culture (100 liters) was filtered in an ordinary manner to separate the cells from the filtrate.

The thus-separated cells (11.3 kg) were suspended in acetone (36 liters), and filtered to obtain an acetone extract. This extract was concentrated under reduced pressure to remove acetone, and the resulting aqueous solution (9 liters) was extracted with 18 liters of ethyl acetate.

The ethyl acetate extract was concentrated under reduced pressure to dryness, and the resulting dry solid (62.77 g) was subjected to silica gel column chromatography (600 g of Kiesel Gel 60, manufactured by Merck Co.) using a developer of n-hexane/acetone (2/1), whereupon were obtained a fraction (0.326 g) containing NA23063B1 and NA23063B2, a fraction (0.301 g) containing NA23063C, and a fraction (0.153 g) containing NA23063D in that order. The thus-obtained fractions were separately purified through ODS column chromatography (Capcell Pak, manufactured by Shiseido Co., with mobile phase of from 60 to 70 % methanol-water) to obtain NA23063B1 (13.5 mg), NA23063B2 (13.5 mg), NA23063C (26.0 mg) and NA23063D (28.0 mg).

In the same manner as above, also obtained was NA23063A (19.5 mg).

These pure NA23063A, NA23063B1, NA23063B2, NA23063C and NA23063D were tested for their appearance, molecular weight, solubility, Rf value in silica gel thin-layer column chromatography, UV absorption spectrum, IR absorption spectrum, ¹H-NMR spectrum, and ¹³C-NMR spectrum. The physicochemical properties of NA23063A, B1, B2, C and D thus obtained have been shown hereinabove.

### INDUSTRIAL APPLICABILITY

The physiologically active substances NA23063 analogues of the invention, especially NA23063A, NA23063B1, NA23063B2, NA23063C and NA23063D have the effect of inhibiting phosphodiesterases, and are expected to be usable as the active ingredient of, for example, antiasthmatic agents, bronchodilators, remedies for bronchitis, antiallergic agents, anti-inflammatory agents, antirheumatic agents, hypotensive drugs, remedies for angina pectoris, remedies for arrhythmia, cerebral vasodilators, blood coagulation inhibiting agents, and antidepressant agents.

## Claims

1. Physiologically active substances, NA23063 analogues of the following formula (1) or their pharmaceutically acceptable salts: 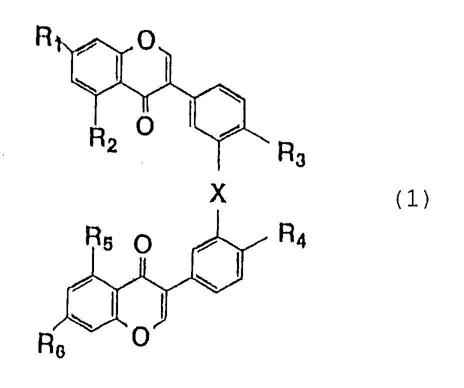 wherein R₁ to R₆ are the same or different substituents, and each independently represent a hydrogen atom, a hydroxyl group or OR; R is one and the same group and represents a C₁₋₄ lower alkyl group, -(CH₂)ₙCOOH, - (CH₂)ₙNYY, a C₁₋₄ lower alkylcarbonyl group, -CO(CH₂)ₙCOOH or -CO(CH₂)ₙNYY; n represents an integer of from 1 to 4; Y represents a hydrogen atom or a C₁₋₄ lower alkyl group, and plural Y's may be the same or different; X represents an oxygen atom or a direct bond; provided that the case where R₁, R₃, R₄ and R₆ are hydroxyl groups, R₂ and R₅ are hydrogen atoms, and X is a direct bond is excluded.

2. Novel compounds of NA23063 analogues as claimed in claim 1, which are as follows:

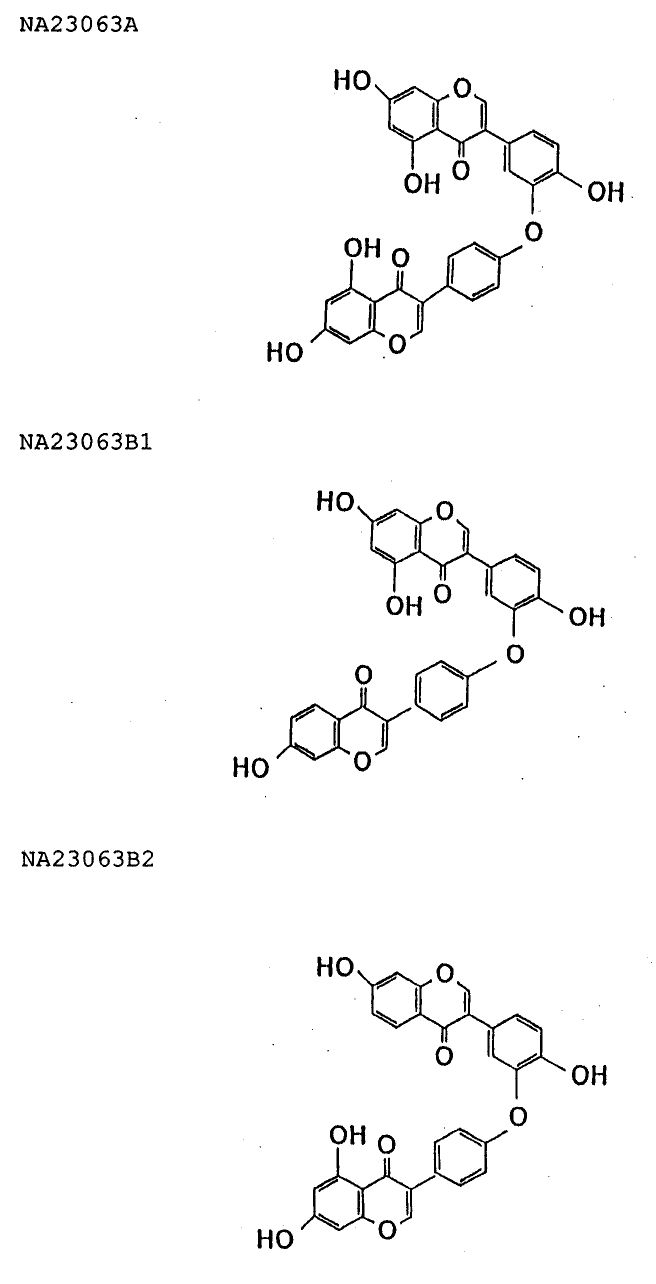

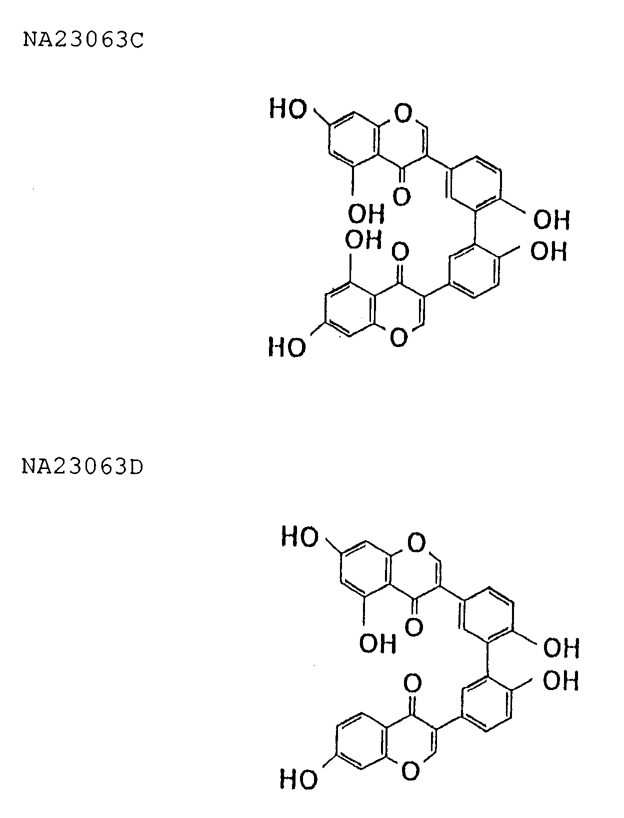

3. A method for producing physiologically active substances, NA23063A, B1, B2, C and D, which comprises cultivating microorganisms belonging to the genus Streptomyces and having the ability of producing physiologically active substances of claim 2, NA23063A, B1, B2, C and D, in a medium to thereby make the microorganisms produce said physiologically active substances NA23063A, B1, B2, C and D, followed by collecting these substances accumulated in the culture.

4. Phosphodiesterase inhibitors comprising, as the active ingredient, any of the physiologically active substances, NA23063 analogues or their pharmaceutically acceptable salts of claim 1 or 2, and containing pharmaceutically acceptable aids.

5. Antiasthmatic agents, bronchodilators, remedies for bronchitis, antiallergic agents, anti-inflammatory agents, antirheumatic agents, hypotensive drugs, remedies for angina pectoris, remedies for arrhythmia, cerebral vasodilators, blood coagulation inhibiting agents, and antidepressant agents, comprising, as the active ingredient, any of the physiologically active substances, NA23063 analogues or their pharmaceutically acceptable salts of claim 1 or 2, and containing pharmaceutically acceptable aids.

6. Phosphodiesterase inhibitors comprising, as the active ingredient, a compound of the following formula: 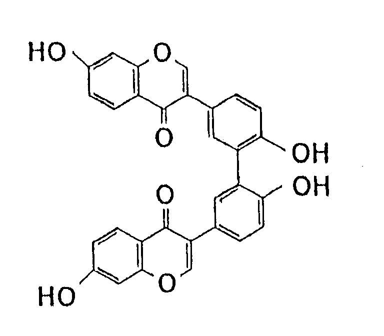 and containing pharmaceutically acceptable aids.

7. Streptomyces sp. NA 23063 having the ability of producing physiologically active substances NA23063A, B1, B2, C and D of claim 2.
